# EUROPEAN PATENT APPLICATION

(11) **EP 4 282 441 A1**
(43) Date of publication of application: **29.11.2023**
(21) Application number: 22742886.9
(22) Date of filing: 21.01.2022
(51) Int. Cl.: A61L 9/20, F24F 8/22, F24F 8/10, B01D 46/00

(54) **FLUID TREATMENT MODULE AND FLUID TREATMENT APPARATUS COMPRISING SAME**

(30) Priority: 22.01.2021 US 202163140497 P
(71) Applicant: Seoul Viosys Co., Ltd., Ansan-si, Gyeonggi-do 15429 (KR)
(72) Inventor: SONG, Jun Ho, Ansan-si Gyeonggi-do 15429 (KR); PARK, Tae Kwan, Ansan-si Gyeonggi-do 15429 (KR); LEE, Dong Jung, Ansan-si Gyeonggi-do 15429 (KR)
(74) Representative: Isarpatent
(86) International application number: PCT/KR2022/001158
(87) International publication number: WO 2022/158910

(57) **Abstract**

The present invention relates to a fluid treatment module and a fluid treatment apparatus comprising same. In particular, according to an embodiment of the present invention, provided is a fluid treatment module comprising: a housing portion having a space formed therein for allowing a fluid to flow in the vertical direction, and having an inlet port for introducing the fluid there and a discharge port for discharging the fluid introduced therein to the outside; a light source portion which can irradiate light to the fluid flowing in the housing portion; and a fluid guide portion which guides the flow of fluid introduced in the housing portion and of which one side is opened in a direction misaligned from the direction in which the inlet port is opened and the other is in communication with the discharge port.

## Description

### TECHNICAL FIELD

The present disclosure relates to a fluid treatment module and a fluid treatment apparatus.

### BACKGROUND ART

Pathogenic infectious agents transmitted in the form of droplets or aerosols are spread by air movement. As the number of cases that the spread of pathogenic infectious agents transmitted in the form of droplets or aerosols increases, a technology capable of sterilizing air circulating indoors is required.

### DETAILED DESCRIPTION OF INVENTION

### TECHNICAL PROBLEMS

In view of the above, one embodiment of the present disclosure provides a fluid treatment apparatus capable of increasing fluid sterilization efficiency by increasing the retention time of the fluid in the fluid treatment apparatus.

### TECHNICAL SOLUTION

In accordance with one aspect of the present disclosure, there is provided a fluid treatment module comprising: a housing part having a space for fluid to flow in an up-down direction therein and having an inlet through which the fluid is introduced into the housing part and an outlet through which the fluid in the housing part is discharged to an outside thereof; a light source unit that irradiates light to the fluid flowing in the housing part; and a fluid guide part that guides the flow of the fluid in the housing part, one end of the fluid guide part being opened in a direction different from a direction in which the inlet is opened, and the other end thereof communicating with the outlet, wherein the inlet is oriented so that the fluid passing through the inlet flows along a direction different from a direction from a center of the inlet to a center of the housing part when viewed from above.

Further, in the fluid treatment module, the outlet may be formed in an upper surface of the housing part, the inlet may be formed in a side surface of the housing part, and the fluid guide part may extend downward from the outlet.

Further, in the fluid treatment module, the fluid guide part may have at the one end a guide opening opened downward, and the guide opening may be disposed at a position higher than 1/2 of a length of the housing part in the up-down direction and lower than or equal to the inlet.

Further, the fluid treatment module may further comprise: a flow path part providing a passage through which the fluid flows and extending from the inlet so that the fluid flows into the housing part, wherein when viewed from above, the flow path part extends in a flow path direction, which is a direction different from the direction from the center of the inlet to the center of the housing part.

Further, in the fluid treatment module, at least a portion of the inlet may overlap the fluid guide part when viewed in the flow path direction.

Further, in the fluid treatment module, the inlet may be disposed to be horizontally spaced apart from an imaginary guide centerline extending in the up-down direction while passing through a center of the fluid guide part when viewed in the flow path direction.

Further, in the fluid treatment module, the fluid guide part and the housing part may be arranged concentrically when viewed from above.

Further, in the fluid treatment module, the housing part may be provided to have a circular or elliptical cross section, a side surface thereof may be extended to have a predetermined inclination with respect to a horizontal direction, and the housing part may be provided so that an upper cross section thereof has a larger cross-sectional area than that of a lower cross section.

Further, the fluid treatment module may further comprise: an adsorption auxiliary part provided on at least one of an inner surface of the housing part and an outer surface of the fluid guide part, wherein the adsorption auxiliary part extends along a circumferential direction of the at least one surface to protrude from or be depressed into the at least one surface.

Further, the fluid treatment module may further comprise: a photocatalyst part provided on at least one of an inner surface of the housing part and an outer surface of the fluid guide part, wherein the photocatalyst part includes at least one of titanium oxide, zinc oxide, and tin oxide that causes a catalytic reaction by the light irradiated from the light source unit.

Further, the fluid treatment module may further comprise: a reflecting part provided on at least one of an inner surface of the housing part and an outer surface of the fluid guide part, wherein the reflecting part includes a reflective material that reflects the light irradiated from the light source unit.

Further, the fluid treatment module may further comprise: an antibacterial part provided on at least one of an inner surface of the housing part and an outer surface of the fluid guide part, wherein the antibacterial part includes an antibacterial material for secondary sterilization of the fluid.

Further, in the fluid treatment module, the fluid guide part may include a transmissive material that transmits the light.

Further, in the fluid treatment module, the light source unit may be disposed between an inner surface of the housing part and an outer surface of the fluid guide part.

Further, there may be provided a fluid treatment module comprising: a housing part having a space for fluid to flow in an up-down direction therein and having an inlet through which the fluid is introduced into the housing part and an outlet through which the fluid in the housing part is discharged to an outside thereof; a light source unit that irradiates light to the fluid flowing in the housing part; and a fluid guide part that guides the flow of the fluid in the housing part, one end of the fluid guide part being opened in a direction different from a direction in which the inlet is opened, and the other end thereof communicating with the outlet, wherein the housing part is provided to have a smaller cross-sectional area from upper side to lower side, and the housing part is configured such that the fluid passing through the inlet swirls along a circumferential direction of the housing part and flows downward.

Further, in the fluid treatment module, the outlet may be formed in an upper surface of the housing part, the inlet may be formed in a side surface of the housing part, and the fluid guide part may be extended downward from the outlet.

Further, there may be provided a fluid treatment apparatus comprising: a fluid treatment module for sterilizing fluid; and a blowing fan for providing a blowing force to cause the fluid to flow into the fluid treatment module, wherein the fluid treatment module includes: a housing part having a space for fluid to flow in an up-down direction therein and having an inlet through which the fluid is introduced into the housing part and an outlet through which the fluid in the housing part is discharged to an outside thereof; a light source unit that irradiates light to the fluid flowing in the housing part; and a fluid guide part that guides the flow of the fluid in the housing part, one end of the fluid guide part being opened in a direction different from a direction in which the inlet is opened, and the other end thereof communicating with the outlet, wherein the inlet is oriented so that the fluid passing through the inlet flows along a direction different from a direction from a center of the inlet to a center of the housing part when viewed from above.

Further, in the fluid treatment apparatus, the fluid treatment module may further include an extension part disposed between the housing part and the blowing fan and guiding a flow of the fluid discharged from the outlet, and the extension part has one end communicating with the outlet and has a shape in which a cross-sectional area becomes wider from the one end to the other end.

Further, the fluid treatment apparatus may further comprise: a filter unit disposed between the extension part and the blowing fan to filter the fluid.

Further, the fluid treatment apparatus may further comprise: a filter unit that filters the fluid, wherein the filter unit includes a plurality of filters disposed adjacent to the inlet and the outlet.

### EFFECT OF INVENTION

According to one embodiment of the present disclosure, the fluid sterilization efficiency can be increased by increasing the retention time of the fluid in the fluid treatment apparatus.

### BRIEF DESCRIPTION OF THE DRAWINGS

FIG. 1 is a front view of a fluid treatment apparatus according to a first embodiment of the present disclosure.
FIG. 2 is a side view of the fluid treatment apparatus of FIG. 1.
FIG. 3 is a cross-sectional view taken along line A-A' of FIG. 1.
FIG. 4 is a cross-sectional view taken along line B-B' of FIG. 1.
FIG. 5 is a plan view of the fluid treatment apparatus of FIG. 1.
FIG. 6 is a longitudinal sectional view of a fluid treatment apparatus according to a second embodiment of the present disclosure.
FIG. 7 is a longitudinal sectional view of a fluid treatment apparatus according to a third embodiment of the present disclosure.
FIG. 8 is a longitudinal sectional view of a fluid treatment apparatus according to a fourth embodiment of the present disclosure.
FIG. 9 is a longitudinal sectional view of a fluid treatment apparatus according to a fifth embodiment of the present disclosure.
FIG. 10 is a longitudinal sectional view of a fluid treatment apparatus according to a sixth embodiment of the present disclosure.
FIG. 11 is a front view of a fluid treatment apparatus according to a seventh embodiment of the present disclosure.
FIG. 12 is a side view of the fluid handling device of FIG. 11.
FIG. 13 is a cross-sectional view taken along line C-C' of FIG. 11.
FIG. 14 is a longitudinal sectional view of a fluid treatment apparatus according to an eighth embodiment of the present disclosure.
FIG. 15 is a longitudinal sectional view of a fluid treatment apparatus according to a ninth embodiment of the present disclosure.
FIG. 16 is a diagram illustrating a state in which fluid flows in the fluid treatment apparatus according to the embodiments of the present disclosure.
FIG. 17 is a diagram illustrating a state in which fluid flows inside the fluid treatment apparatus in which a fluid guide part is omitted.

### MODE FOR CARRYING OUT THE INVENTION

Hereinafter, specific embodiments for implementing the technical idea of the present disclosure will be described in detail with reference to the drawings.

In addition, in the description of the present disclosure, when it is determined that a detailed description of a related known configuration or function may obscure the gist of the present disclosure, the detailed description will be omitted.

In addition, when a component is referred to as being 'connected', 'supported', ' introduced', 'discharged', or 'moved' to another component, it should be understood that the component may be connected, supported, introduced, discharged, or moved directly to another component, but other components may exist therebetween.

Terms used in the present specification are only used to describe the specific embodiments and are not intended to limit the present disclosure. Singular expressions include plural expressions unless the context clearly indicates otherwise.

In addition, terms including ordinal numbers, such as first, second, etc., may be used to describe various components, but the components are not limited by these terms. These terms are only used to distinguish one component from another.

As used herein, the meaning of "including" specifies specific characteristics, regions, integers, steps, operations, elements and/or components, and does not exclude the presence or addition of other specific characteristics, regions, integers, steps, operations, elements, components and/or groups.

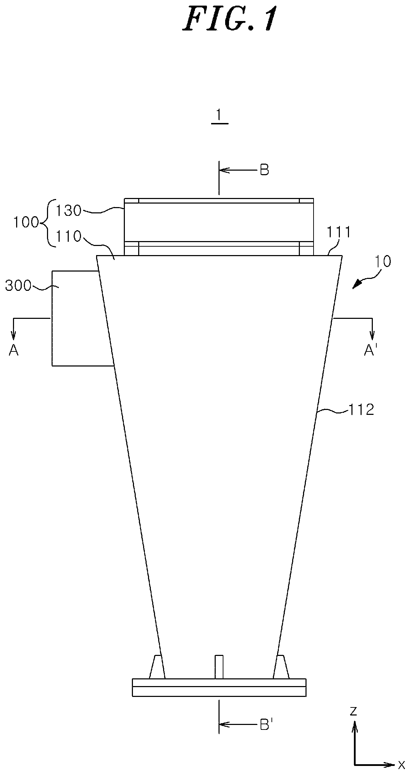

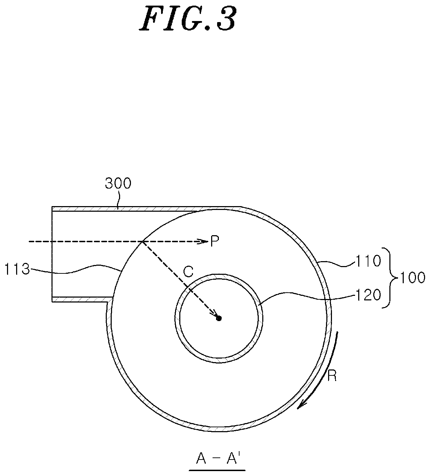

Further, in the present specification, expressions for directions such as upper, lower, and the like are described based on the drawings, and it is made clear in advance that they may be expressed differently when the direction of an object is changed. Meanwhile, in the present specification, an up-down direction may be the z-axis direction of FIG. 1, and a horizontal direction may be a direction perpendicular to the z-axis direction of FIG. 1 (e.g., the x-axis direction of FIG. 1). Furthermore, a circumferential direction may be the circumferential direction R of a housing part 110 of FIG. 3.

Hereinafter, a specific configuration of a fluid treatment apparatus 1 according to one embodiment of the present disclosure will be described with reference to the drawings.

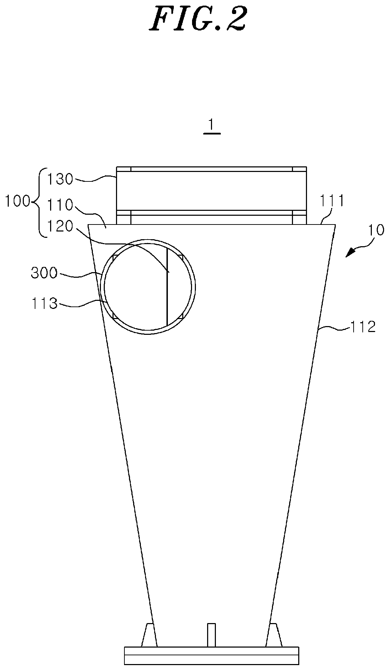

Referring to FIGS. 1 to 4, a fluid treatment apparatus 1 according to one embodiment of the present disclosure can treat fluid flowing into the fluid treatment apparatus 1. In the present specification, the fluid to be treated by the fluid treatment apparatus 1 may be water or air, and the treatment of the fluid by the fluid treatment apparatus 1 may include sterilization, purification, and deodorization of droplets included in the fluid by providing light to the fluid. In this case, the fluid treatment apparatus 1 may be referred to as a fluid sterilization device, a fluid purification device, a fluid deodorization device, and a light providing device. For example, droplets included in the fluid may be bacteria, viruses, and fine dust harmful to the human body, and may have a size of 3 µm to 10 µm. In this case, the fluid treatment apparatus 1 may treat and kill bacteria, viruses, and the like.

In addition, the fluid treatment apparatus 1 may adsorb droplets included in the fluid from the fluid. For example, the fluid treatment apparatus 1 may provide clean fluid to a user by adsorbing droplets included in the fluid to an inner surface of the fluid treatment apparatus 1 or filtering out them from the fluid. The fluid treatment apparatus 1 may include a fluid treatment module 10 and a blowing fan 20.

The fluid treatment module 10 may sterilize the fluid that is introduced into the fluid treatment module 10 from the outside and flows inside the fluid treatment module 10. In addition, the fluid treatment module 10 may accommodate the blowing fan 20, and the fluid may be introduced into the fluid treatment module 10 by a blowing force provided from the blowing fan 20. The fluid treatment module 10 may include a body 100, a light source unit 200, and a flow path part 300.

The body 100 may support the light source unit 200 and the flow path part 300, and may provide a space for fluid to flow therein. In addition, the body 100 may guide the flow of the fluid. The body 100 may include a housing part 110, a fluid guide part 120, and a support part 130.

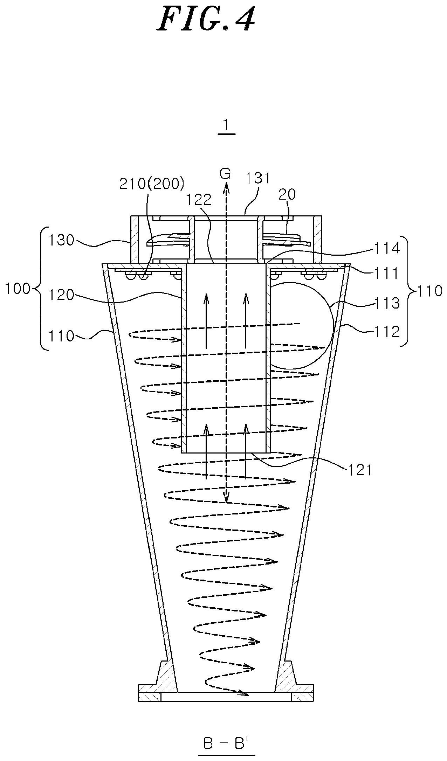

Referring to FIGS. 3 and 4, the housing part 110 may provide a space for fluid to flow in an up-down direction therein, and may provide a space for sterilizing the fluid. The housing part 110 may be configured such that the fluid passing through an inlet 113 to be described later swirls along a circumferential direction of the housing part 110 and flows downward. In this case, the swirling of the fluid means that the fluid spirally flows along the circumferential direction of the housing part 110 like a vortex.

For example, the housing part 110 may have a circular or elliptical cross section. In this case, the cross section of the housing part 110 means a cross section obtained by cutting the housing part 110 along a horizontal direction (perpendicular to the up-down direction). In addition, the housing part 110 may be provided so that its cross-sectional area becomes smaller from the top to the bottom. In other words, the upper cross-sectional area of the housing part 110 may be larger than the lower cross-sectional area. In this case, the housing part 110 may be provided in an upside-down inverted conical shape. In other words, the housing part 110 may have a tapered cylindrical shape with a narrower lower portion. However, this is only an example, and the housing part 110 may be provided in a cylindrical shape, or may be provided in a polygonal shape such as a square in cross section.

Since the housing part 110 is configured to have different cross-sectional areas in the up-down direction, the flow speed of the fluid may be different depending on the position of the fluid. For example, the flow speed of the fluid flowing along the inner surface of the housing part 110 may be slower in the upper side with the greater cross-sectional area than in the lower side of the housing part 110. In addition, since the housing part 110 is configured to have different cross-sectional areas in the up-down direction, the fluid flowing inside the housing part 110 may form a vortex. In this case, retention time of the fluid introduced into the housing part 110 can be increased. In the present specification, the retention time of the fluid means the time during which the fluid introduced into the housing part 110 stays inside the housing part 110 housing part 110 until it is discharged to the outside of the housing part 110.

Meanwhile, the housing part 110 may have a upper surface 111, a side surface 112, an inlet 113, and an outlet 114.

The upper surface 111 of the housing part 110 may support the light source unit 200. The upper surface 111 of the housing part 110 may extend in the horizontal direction. In addition, the upper surface 111 of the housing part 110 may support the support part 130.

The side surface 112 of the housing part 110 may support the flow path part 300. The side surface 112 of the housing part 110 may be disposed to be inclined at a predetermined angle with respect to the upper surface 111 of the housing part 110. In other words, the side surface 112 of the housing part 110 may extend along a direction different from the horizontal direction. For example, the side surface 112 of the housing part 110 may extend such that an upper circumference is longer than a lower circumference. In this case, the fluid passing through the inlet 113 flows downward along the inner surface of the housing part 110, and the retention time of the fluid becomes longer. Meanwhile, in the present specification, a surface facing the inner space of the housing part 110 among the side surfaces 112 of the housing part 110 may be referred to as the inner surface of the housing part 110.

The inner surface of the housing part 110 may be provided to have a predetermined roughness to adsorb droplets included in the fluid. For example, droplets included in the fluid flowing inside the housing part 110 may be adsorbed to the inner surface of the housing part 110 while swirling along the inner surface of the housing part 110.

Due to such a shape of the housing part 110, the time for the fluid to flow along the inner surface of the housing part 110 increases, and more droplets are adsorbed to the inner surface of the housing part 110. In this case, the droplet removal efficiency of the fluid treatment apparatus 1 is improved.

The inlet 113 may be provided so that fluid flowing along the flow path part 300 is introduced into the housing part 110. The inlet 113 may communicate the inside of the housing part 110 and the flow path part 300. In addition, the inlet 113 may be oriented so that the fluid passing through the inlet 113 flows in a direction different from a direction from the center of the inlet 113 toward the center of the housing unil110 when viewed from above. In other words, the inlet 113 may be oriented so that the fluid passing through the inlet 113 flows along a flow direction P that is different from a central direction C when viewed from above.

Referring back to FIG. 3, in the present specification, the central direction C refers to a direction in which an imaginary line connecting the center of the inlet 113 and the center of the housing part 110 extends when viewed from above. In addition, the flow direction P means a direction that is different from the central direction C when viewed from above, and means a direction in which the flow path part 300 extends. For example, the flow rate of the fluid passing through the inlet 113 along the flow direction P is greater than the flow rate along the central direction C. In this case, the fluid passing through the inlet 113 flows along the inner surface of the housing part 110 in a circumferential direction R of the housing part 110.

Meanwhile, referring to FIG. 4, the inlet 113 may be formed on the side surface 112 of the housing part 110 and may be disposed at the upper side of the housing part 110. For example, the inlet 113 may be disposed at a position higher than a point corresponding to 1/2 of a length of the housing part 110 in the up-down direction. The inlet 113 may overlap the fluid guide part 120 when viewed in the flow direction P. In addition, the inlet 113 may be disposed to be horizontally spaced apart from an imaginary guide center line G extending in the up-down direction while passing through the center of the fluid guide part 120. In this case, the separation of the inlet 113 from the guide center line G may be a concept including the separation of the center of the inlet 113 from the guide center line G.

The outlet 114 may be provided to discharge the fluid introduced into the housing part 110 to the outside of the housing part 110. The outlet 114 communicates with the fluid guide part 120, and allows the fluid guide part 120 to communicate with the outside of the housing part 110. In addition, the outlet 114 may be formed in the upper surface 111 of the housing part 110.

The fluid guide part 120 may guide the flow of fluid introduced into the housing part 110. The fluid guide part 120 may have a first guide opening 121 and a second guide opening 122. The first guide opening 121 may be opened in a direction different from the direction in which the inlet 113 is opened to prevent the fluid passing through the inlet 113 from directly flowing into the fluid guide part 120. For example, the first guide opening 121 may be opened downward. In this case, the fluid passing through the inlet 113 does not flow directly into the fluid guide part 120 but flows downward.

In addition, the fluid guide part 120 may extend such that an outer surface of the fluid guide part 120 faces the inlet 113. For example, the fluid guide part 120 is supported on the upper surface 111 of the housing part 110 and may extend downward from the upper surface 111. In this case, the fluid passing through the inlet 113 collides with the outer surface of the fluid guide part 120. In addition, the fluid passing through the inlet 113 does not directly flow into the fluid guide part 120 but flows downward along the inner surface of the housing part 110.

For example, the fluid guide part 120 guides the fluid introduced into the housing part 110 to flow upward after flowing downward. In this case, the retention time of the fluid and the time during which the fluid is irradiated with light increase, and the efficiency of sterilization, deodorization, and purification of the fluid increases.

The second guide opening 122 is connected to the upper surface 111 of the housing part 110 and may communicate with the outlet 114. For example, the fluid introduced into the first guide opening 121 may pass through the second guide opening 122 and the outlet 114 sequentially to be discharged to the outside of the housing part 110. In addition, a width of the second guide opening 122 may be smaller than that of the first guide opening 121. In this case, a bottleneck phenomenon occurs while the fluid flows from the first guide opening 121 toward the second guide opening 122, which decreases the flow speed of the fluid and increases the retention time of the fluid. In other words, the fluid treatment efficiency of the fluid treatment apparatus 1 is increased.

Meanwhile, the fluid guide part 120 may be provided in a cylindrical shape having a circular cross section, and when viewed from above, may be placed concentrically with the housing part 110. In other words, the center of the fluid guide part 120 may coincide with the center of the housing part 110. The fluid guide part 120 may be disposed so that the position of the first guide opening 121 is lower than or the same as the position of the inlet 113. In this case, the position lower than or the same as the inlet 113 may mean a position lower than a lower end of the inlet 113. For example, the fluid guide part 120 may be provided so that the inlet 113 is placed at a point adjacent to the second guide opening 122 among points that are 1/3 of a length of the fluid guide part 120. In other words, the distance from the first guide opening 121 to a central axis of the inlet 113 is twice the distance from the second guide opening 122 to the central axis of the inlet 113.

In addition, the fluid guide part 120 may be disposed such that the first guide opening 121 is placed at a position higher than the point that is 1/2 of the length of the housing part 110 and lower than the point that is 2/5 of the length of the housing part 110 in the up-down direction. In this case, the flow speed of the fluid flowing on the upper side of the first guide opening 121 may be slower than the flow speed of the fluid flowing on the lower side of the first guide opening 121.

For example, when the fluid guide part 120 is disposed such that the first guide opening 121 is placed at a position lower than a point that is 1/2 of the length of the housing part 110 in the up-down direction, the distance between the bottom of the housing part 110 and the first guide opening 121 is shortened. In this case, the fluid flowing in the lower side of the housing part 110 directly flows into the first guide opening 121, and the retention time of the fluid is shortened. As another example, when the fluid guide part 120 is disposed such that the first guide opening 121 is placed at a position higher than a point adjacent to the second guide opening 121 among points that are 2/5 of the length of the housing part 110 in the up-down direction, the distance between the inlet 113 and the first guide opening 121 is shortened. In this case, the fluid passing through the inlet 113 directly flows into the first guide opening 121, and the retention time of the fluid is shortened.

Due to such arrangement and length of the fluid guide part 120, the retention time of the fluid increases and the time during which the fluid is irradiated with light also increases. In addition, the fluid sterilizing effect, the fluid deodorizing effect, and the fluid purifying effect of the fluid treatment apparatus 1 are increased.

Meanwhile, in the present specification, a side surface of the fluid guide part 120 facing the side surface 112 of the housing part 110 may be referred to as the outer surface of the fluid guide part 120. In addition, an imaginary line passing through the center of the fluid guide part 120 and extending in the up-down direction may be referred to as the guide center line G (see FIG. 4).

The retention time (sec) of the fluid and the residual rate (%) of the droplets in the housing part 110 depending on the inclination of the side surface 112 of the housing part 110 and the presence or absence of the fluid guide part 120 are shown in Table 1 below.

**(Table 1)**

| | Inclination of side surface of housing part | Fluid guide part | Retention time of fluid (sec) | Residual rate of droplets in housing part (%) |
|---|---|---|---|---|
| Embodiment | Presence | Presence | 0.372 | 28 |
| Comparative Example | Absence | Absence | 0.2 | 8.5 |

As shown in Table 1 above, in the embodiment of the present disclosure, the retention time is increased by about 1.9 times and the droplet residual rate by about 3.3 times compared to the Comparative Example. According to the embodiment of the present disclosure, the retention time of the fluid in the housing part 110 is increased, so more droplets are removed from the fluid and adsorbed to the inner surface of the housing part 110. In addition, the time during which the droplets adsorbed on the inner surface of the housing part 110 are irradiated with light increases, so that the sterilization efficiency also increases.

The support part 130 is supported on the upper surface 111 of the housing part 110 and can support the blowing fan 20. In addition, the support part 130 may be provided to surround at least a portion of the blowing fan 20. For example, the support part 130 may have the outlet131 at least partially opened so that fluid can pass therethrough.

The light source unit 200 may irradiate light to the fluid flowing in the body 100. The light source unit 200 may irradiate ultraviolet light by adjusting the wavelength of light according to the type of fluid to be sterilized and the type of target (e.g., virus, mold, dust, etc.). For example, the light source unit 200 may irradiate light in an ultraviolet wavelength band when sterilizing a target in the fluid, and the light source unit 200 may emit ultraviolet light in a wavelength range of 200 nm to 405 nm capable of sterilizing bacteria or viruses. These ultraviolet rays can be classified into UV-A with a wavelength of 320 nm to 400 nm, UV-B with a wavelength of 280 nm to 320 nm, and UV-C with a wavelength of 200 nm to 280 nm. As a more specific example, the light source unit 200 may emit UV-C light having the wavelength of 200 nm to 280 nm. Such UV-C light has a high sterilizing power, and ultraviolet rays at an intensity of 100 µW per 1 cm² can kill about 99% of bacteria such as Escherichia coli, diphtheria, and dysentery.

In addition, UV-C light can kill bacteria that cause food poisoning, and, for example, can kill bacteria such as pathogenic Escherichia coli, Staphylococcus aureus, Salmonella Weltevreden, Salmonella Typumurium, Enterococcus faecalis, Bacilluscereus, Pseudomonas aeruginosa, Vibrio parahaemolyticus, Listeria monocytogenes, Yersinia enterocolitica, Clostridium botulinum, Campylobacter jejuni, Enterobacter sakazakii, or the like that causes food poisoning.

Further, UV-C light can sterilize SARS coronavirus (SARS CoV), novel swine-origin influenza A (H1N1), influenza virus, mumps virus, Rubella virus, and the like that spread through droplets and the air to cause disease.

Meanwhile, the light source unit 200 may include one or more light sources 210. When a plurality of light sources 210 are provided, the plurality of light sources 210 may emit light of the same wavelength band or may emit lights of different wavelengths, respectively. In addition, one or more light sources 210 may be provided on the upper surface 111 of the housing part 110 and may emit light downward. In this case, light irradiated from the light source 210 may be prevented from being exposed to the outside through the outlet 114.

In addition, the light source 210 may be disposed between the housing part 110 and the fluid guide part 120. For example, the light source 210 may be disposed between the inner surface of the housing part 110 and the outer surface of the fluid guide part 120 in the horizontal direction, and may be disposed at a middle point between the inner surface of the housing part 110 and the outer surface of the fluid guide part 120. As a more specific example, the light source 210 may be disposed adjacent to the inlet 113. In this case, due to the flow of fluid introduced from the inlet 113, overheating of the light source 210 can be prevented, and light output performance degradation of the light source 210 can be prevented. In addition, when a plurality of light sources 210 are provided, the plurality of light sources 210 may be spaced apart along the circumferential direction. For example, the light source 210 may include a light emitting diode device. Meanwhile, the light source unit 200 may further include a printed circuit board (not shown) on which the light source 210 including the light emitting diode is disposed.

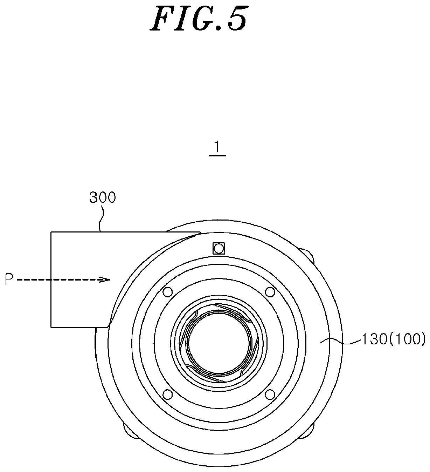

Referring to FIGS. 3 to 5, the flow path part 300 may provide a passage through which fluid flows. The flow path part 300 may extend from the inlet 113 so that fluid flows into the housing part 110. For example, the fluid flowing along the flow path part 300 may pass through the inlet 113 and flow into the housing part 110. In addition, the flow path part 300 may determine the flow direction of the fluid when the fluid flows into the housing 110. In other words, the flow direction of the fluid that has sequentially passed through the flow path part 300 and the inlet 113 is determined by the extension direction of the flow path part 300. For example, when viewed from above, the flow path part 300 may extend in a direction different from the direction from the center of the inlet 113 toward the center of the housing 110. In other words, when viewed from above, the flow path part 300 may extend along the flow direction P that is different from the central direction C.

Meanwhile, when viewed in the flow direction P, the flow path part 300 may overlap the fluid guide part 120. In addition, when viewed in the flow direction P, the flow path part 300 may be disposed to be spaced apart from the guide center line G of the fluid guide part 120 in the horizontal direction. In other words, the flow direction P of the flow path part 300 does not intersect with the central axis of the fluid guide part 120 not to be directed to the central axis of the fluid guide part 120. When viewed from above, the flow path part 300 is disposed offset from the central axis of the fluid guide part 120. In this case, the flow direction of the fluid passing through the flow path part 300 is easily changed even with a low hydraulic pressure, and flows along the inner surface of the housing part 110 around the fluid guide part 120. By the flow path part 300 and the fluid guide part 120, the fluid flowing in the housing part 110 flows along a longer flow path, and the retention time of the fluid increases. In other words, the fluid treatment efficiency of the fluid treatment apparatus 1 can be increased.

The blowing fan 20 may be driven so that fluid outside the fluid treatment module 10 flows into the fluid treatment module 10. The blowing fan 20 may provide blowing force so that the introduced fluid is discharged to the outside. Further, the blowing fan 20 may be supported by the support part 130. Although the blowing fan 20 is illustrated as being disposed adjacent to the outlet 114 in the present specification, this is only an example, and the blowing fan 20 may be disposed adjacent to the inlet 113.

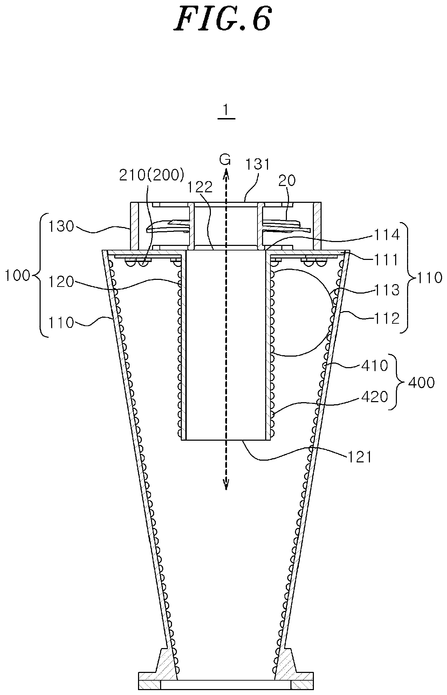

Meanwhile, in addition to such configurations, according to a second embodiment of the present disclosure, the fluid treatment module 10 may further include an adsorption auxiliary part 400. Hereinafter, the second embodiment of the present disclosure will be described with further reference to FIG. 6. In describing the second embodiment, differences compared with the above-described embodiment are mainly described, and the same description and reference numerals are referred to the above-described embodiment.

The adsorption auxiliary part 400 may assist bacteria and dust particles included in the fluid to be better adsorbed to the inner surface of the housing part 110 and the outer surface of the fluid guide part 120. For example, the adsorption auxiliary part 400 may be formed as a protrusion, a groove, or the like, and may be provided in a hemisphere shape. In addition, the absorption auxiliary part 400 may include a reflective material capable of reflecting light emitted from the light source unit 200. The reflective material may include one or more of highly reflective stainless steel, aluminum and magnesium oxide. The adsorption auxiliary part 400 may include a first adsorption auxiliary part 410 and a second adsorption auxiliary part 420.

The first suction auxiliary part 410 may protrude from the inner surface of the housing part 110 toward the inner space of the housing part 110. The first suction auxiliary part 410 may be provided in plurality and may be provided along the side surface 112 of the housing part 110.

The second suction auxiliary part 420 may protrude from the outer surface of the fluid guide part 120 toward the side surface 112 of the housing part 110. The second suction auxiliary part 420 may be provided in plural and may be provided along the side surface of the fluid guide part 120.

Although the adsorption auxiliary part 400 has been described as being provided on both the inner surface of the housing part 110 and the outer surface of the fluid guide part 120 in the present specification, this is only an example and the present disclosure is not limited thereto. Therefore, the adsorption auxiliary part 400 may not be provided on the outer surface of the fluid guide part 120 and may be provided only on the inner surface of the housing part 110. In addition, the adsorption auxiliary part 400 may not be provided on the inner surface of the housing part 110 and may be provided only on the outer surface of the fluid guide part 120.

The adsorption auxiliary part 400 according to the second embodiment can increase the adsorption rate of droplets included in the fluid and increase the retention time of the droplets. In this case, the time during which the light is irradiated to the droplets increases, and the fluid sterilization effect, the fluid deodorization effect, and the fluid purification effect can be increased.

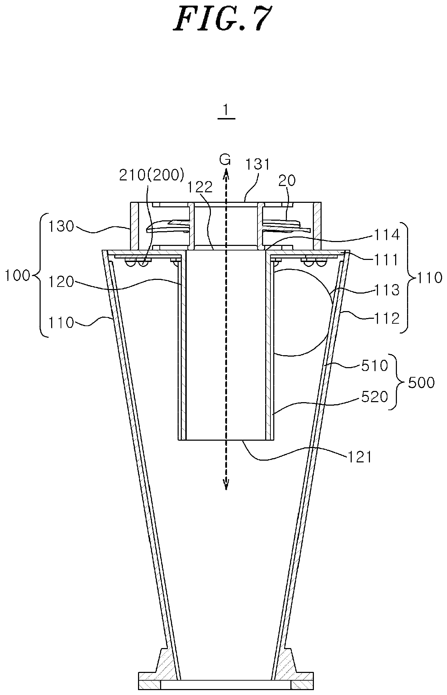

Meanwhile, according to a third embodiment of the present disclosure, the fluid treatment module 10 may further include a photocatalyst part 500. Hereinafter, the third embodiment of the present disclosure will be described with further reference to FIG. 7.

The photocatalyst part 500 may cause a catalytic reaction by light irradiated from the light source unit 200. The photocatalyst part 500 is activated by light irradiated from the light source unit 200 and causes a chemical reaction to sterilize a target in the fluid contacting the photocatalyst part 500. For example, the photocatalyst part 500 causes a chemical reaction in which electrons and holes are generated when light having a predetermined band gap energy or more is irradiated from the light source unit 200. In this case, hydroxyl radicals and superoxide ions are generated from water and oxygen in the air. These hydroxyl radicals and superoxide ions generate a deodorizing effect by decomposing organic contaminants in the fluid, and sterilize contaminants such as bacteria by inactivating the contaminants in the fluid.

The photocatalytic part 500 may include a photocatalytic material that causes a photocatalytic reaction by ultraviolet rays, and may include, for example, one or more of titanium oxide (TiO₂), zinc oxide (ZnO), and tin oxide (SnO₂). In addition, since holes and electrons generated on the surface of the photocatalyst part 500 have a fast recombination rate, a metal such as Pt, Ni, Mn, Ag, W, Cr, Mo, Zn, or an oxide thereof may be added to delay the recombination rate of the holes and electrons. The delay in the recombination rate of the holes and electrons increases the likelihood of contact with bacteria in the fluid to be oxidized and/or decomposed, which may increase the reactivity.

As a more specific example, the photocatalyst part 500 may include titanium oxide (TiO₂). When the titanium oxide is irradiated with ultraviolet light having a wavelength of 400 nm or less, the titanium oxide can generate superoxygen radicals, which decompose organic matter into water and carbon dioxide. The titanium oxide has excellent decomposition ability, has continuous durability and stability even in environmental changes, and has a semi-permanent effect. The titanium oxide may be more active in UV-A of the wavelength of 320 nm to 400 nm, and the one or more light sources 210 may irradiate UV-A.

The photocatalyst part 500 may be applied to the inner surface of the housing part 110 and the outer surface of the fluid guide part 120. In addition, the photocatalyst part 500 may include a first photocatalyst part 510 and a second photocatalyst part 520.

The first photocatalyst part 510 may be provided on the inner surface of the housing part 110, and the second photocatalyst part 520 may be provided on the outer surface of the fluid guide part 120.

Although the photocatalyst part 500 has been described as being provided on both the inner surface of the housing part 110 and the outer surface of the fluid guide part 120 in the present specification, this is only an example and the present disclosure is not limited thereto. Accordingly, the photocatalyst part 500 may not be provided on the outer surface of the fluid guide part 120, and may be provided only on the inner surface of the housing part 110. In addition, the photocatalyst part 500 may not be provided on the inner surface of the housing part 110, and may be provided only on the outer surface of the fluid guide part 120.

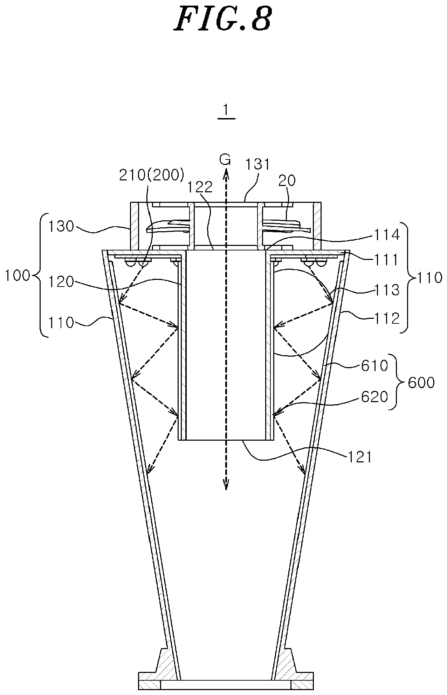

Meanwhile, according to a fourth embodiment of the present disclosure, the fluid treatment module 10 may further include a reflecting part 600. Hereinafter, the fourth embodiment of the present disclosure will be described with further reference to FIG. 8.

The reflecting part 600 may reflect light emitted from the light source unit 200. The reflecting part 600 may reflect the light emitted from the light source unit 200 so that the light reaches an area inside the housing part 110 where light does not reach. Further, the reflecting part 600 may reflect the light emitted from the light source unit 200 multiple times. In this case, the number of times the light irradiated from the light source unit 200 reaches the fluid also increases, so the fluid sterilization efficiency can increase. The reflecting part 600 may include a reflective material having high reflectivity. For example, the reflecting part 600 may include a reflective material such as aluminum (Al), stainless steel, or magnesium oxide, but the reflecting part 600 is not limited thereto, and various materials may be used as long as they are capable of reflecting light. In addition, the reflecting part 600 may be applied to the inner surface of the housing part 110 and the outer surface of the fluid guide part 120 or may be provided as a separate member. The reflecting part 600 may include a first reflecting part 610 and a second reflecting part 620.

The first reflecting part 610 may be provided on the inner surface of the housing part 110 and may reflect light irradiated onto the inner surface of the housing part 110. In addition, the second reflecting part 620 may be provided on the outer surface of the fluid guide part 120 and may reflect light irradiated onto the outer surface of the fluid guide part 120.

Although the reflecting part 600 has been described as being provided on both the inner surface of the housing part 110 and the outer surface of the fluid guide part 120 in the present specification, this is only an example and the present disclosure is not limited thereto. Therefore, the reflecting part 600 may not be provided on the outer surface of the fluid guide part 120 and may be provided only on the inner surface of the housing part 110. In addition, the reflecting part 600 may not be provided on the inner surface of the housing part 110 and may be provided only on the outer surface of the fluid guide part 120.

Further, in the fluid treatment module 10 according to the fourth embodiment, the reflecting part 600 may not be separately provided, and the housing part 110 and the fluid guide part 120 may be formed of a material having high reflectivity.

The reflecting part 600 according to the fourth embodiment can reflect the light irradiated from the light source unit 200 multiple times, which increases the time during which the fluid is irradiated with the light. In this case, the fluid sterilization effect, the fluid deodorization effect, and the fluid purification effect can be increased.

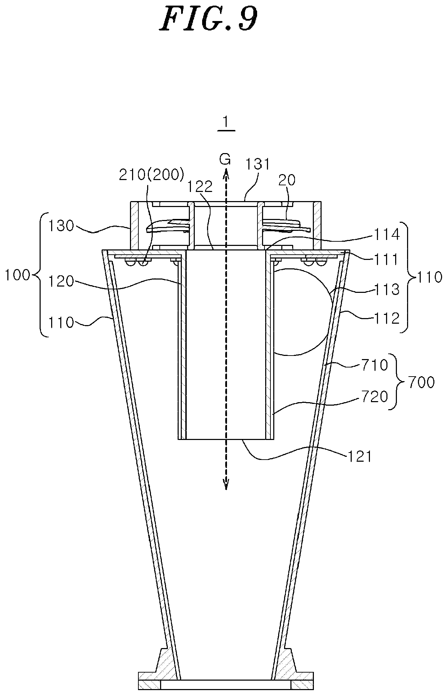

Meanwhile, according to a fifth embodiment of the present disclosure, the fluid treatment module 10 may further include an antibacterial part 700. Hereinafter, the fifth embodiment of the present disclosure will be described with further reference to FIG. 9.

The antibacterial part 700 can prevent bacteria and viruses from propagating by sterilizing bacteria and viruses remaining inside the body 100. In order to prevent bacteria and viruses adsorbed on the inner surface of the housing part 110 and the outer surface of the fluid guide part 120 from propagating, the antibacterial part 700 may be provided on the inner surface of the housing part 110 and the outer surface of the fluid guide part 120. In addition, the antibacterial part 700 may be provided on the inner surface of the housing part 110 and the outer surface of the fluid guide part 120. For example, the antibacterial part 700 may be a powder containing an antibacterial substance, a film containing an antibacterial substance, or the like. In addition, the antibacterial material may be metal nanoparticles, and the metal nanoparticles may include one or more of silver (Ag), bronze, brass, nickel (Ni), aluminum (Al), tin (Sn), and zinc (Zn). The antibacterial part 700 may include a first antibacterial part 710 and a second antibacterial part 720.

The first antibacterial part 710 may be provided on the inner surface of the housing part 110, and the second antibacterial part 720 may be provided on the outer surface of the fluid guide part 120.

Although the antibacterial part 700 has been described as being provided on both the inner surface of the housing part 110 and the outer surface of the fluid guide part 120 in the present specification, this is only an example and the present disclosure is not limited thereto. Accordingly, the antibacterial part 700 may not be provided on the outer surface of the fluid guide part 120 and may be provided only on the inner surface of the housing part 110. In addition, the antibacterial part 700 may not be provided on the inner surface of the housing part 110 and may be provided only on the outer surface of the fluid guide part 120.

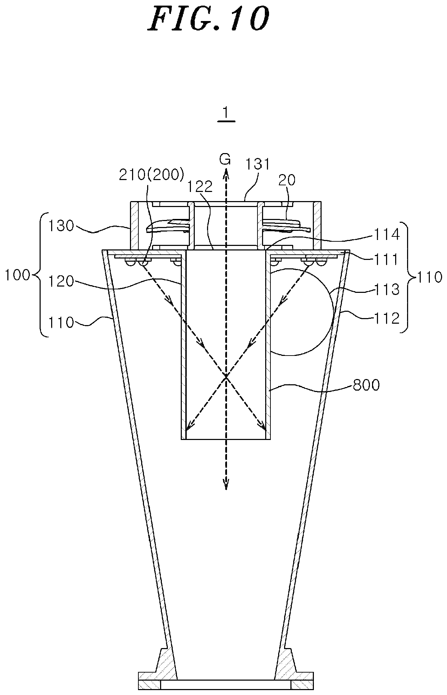

Meanwhile, according to a sixth embodiment of the present disclosure, the fluid guide part 120 may be formed of a permeable material. Hereinafter, the sixth embodiment of the present disclosure will be described with further reference to FIG. 10, and the aforementioned fluid guide part 120 may be referred to as a transmissive guide part 800 in the sixth embodiment.

The transmissive guide part 800 may transmit light emitted from the light source unit 200. In this case, the transmissive guide part 800 transmitting light means that the transmissive guide part 800 transmits light having a predetermined range of wavelengths, but does not mean that the transmissive guide part 800 is transparent when viewed with the naked eye. Therefore, when viewed with the naked eye, the transmissive guide part 800 may be opaque. The transmissive guide part 800 may transmit the light emitted from the light source part 200 so that the light emitted from the light source part 200 can reach the fluid flowing in the transmissive guide part 800.

In addition, the transmissive guide part 800 may include a transmissive material that transmits UV wavelengths irradiated from the light source unit 200. For example, the transmissive guide part 800 may include quartz or a polymeric organic material. As a more specific example, the polymeric organic material may include at least one of polyvinylalcohol (PVA), polypropylene (PP), and low-density polyethylene (PE), which are capable of transmitting ultraviolet rays.

The transmissive guide part 800 according to the sixth embodiment can transmit light. In this case, the light irradiated from the light source unit 200 reaches the fluid flowing in the transmissive guide part 800, so that the fluid sterilization efficiency can be increased.

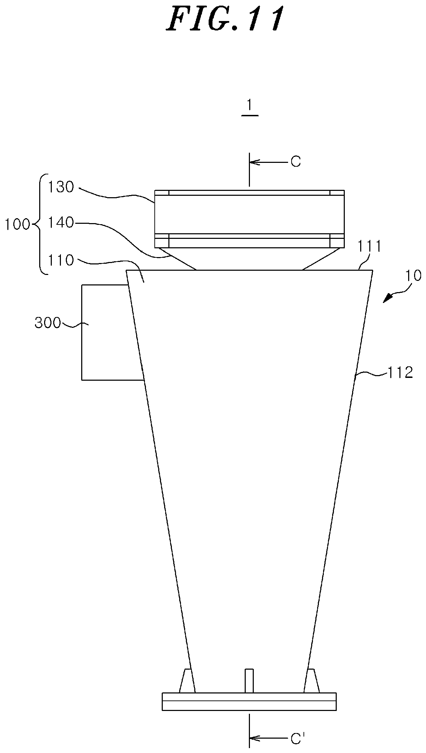

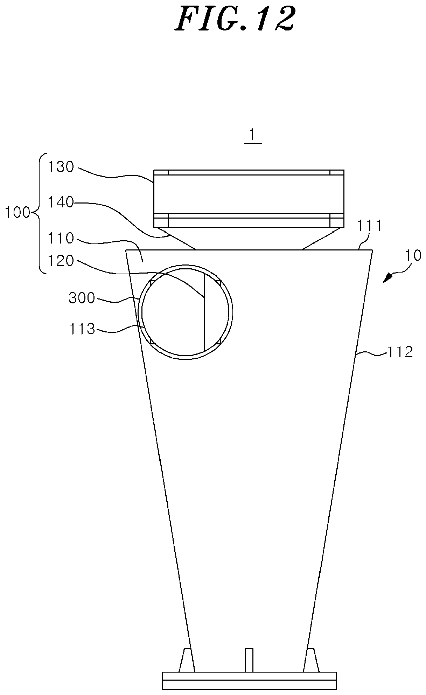

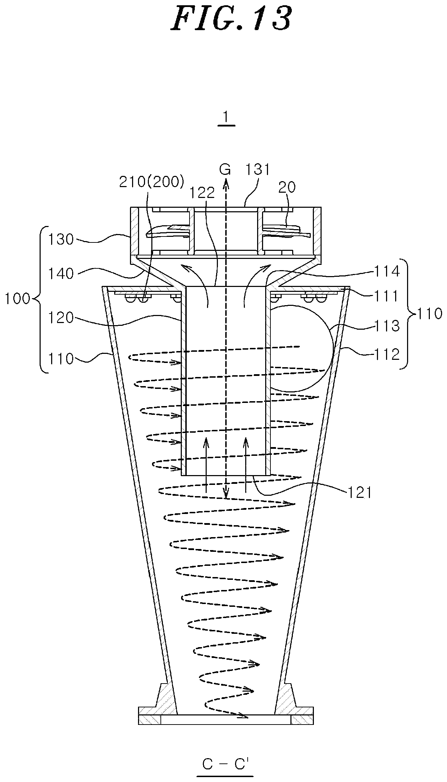

Meanwhile, according to a seventh embodiment of the present disclosure, the fluid treatment module 10 may further include an extension part 140. Hereinafter, the seventh embodiment of the present disclosure will be described with further reference to FIGS. 11 to 13.

The body 100 may further include the extension part 140. The extension part 140 may guide the flow of the fluid discharged through the outlet 114 and assist the fluid discharged through the outlet 114 to flow more easily. For example, one side of the extension part 140 may be connected to the outlet 114 and the other side may be connected to the support part 130. In addition, the extension part 140 may have a shape in which a cross-sectional area increases from one side connected to the outlet 114 to the other side connected to the support 130. In this case, the fluid passing through the discharge port 114 can more smoothly flow toward the blowing fan 20. Meanwhile, the extension part 140 may be disposed between the housing part 110 and the support part 130. For example, the extension part 140 may be disposed above the outlet 114 and below the blowing fan 20.

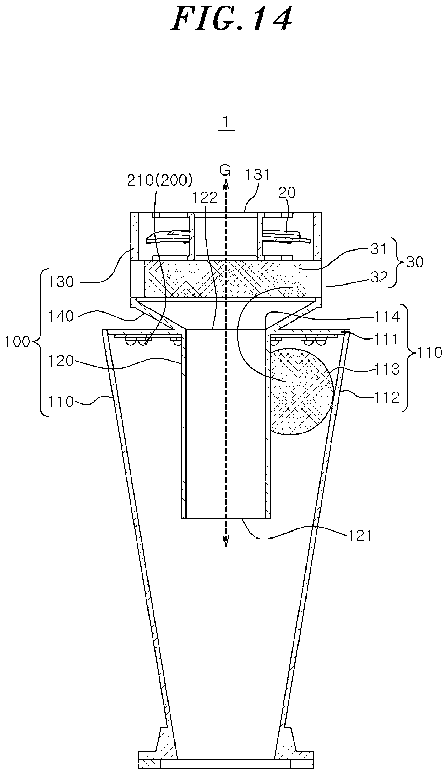

Meanwhile, according to an eighth embodiment of the present disclosure, the fluid treatment apparatus 1 may further include a filter unit 30. Hereinafter, the eighth embodiment of the present disclosure will be described with further reference to FIG. 14.

The filter unit 30 may filter out particles such as bacteria, viruses, and fine dust included in the fluid. The filter unit 30 may include various filters according to purposes, and may include, for example, one or more of a HEPA filter, a carbon filter, and a photocatalyst filter. In this case, the filter unit 30 may kill bacteria and viruses included in the fluid or filter out particles such as fine dust. As a more specific example, when the filter unit 30 includes the HEPA filter, organic or inorganic particles of 0.3 µm or less may be filtered out. In addition, when the filter unit 30 includes the photocatalyst filter, bacteria or the like that come into contact with the photocatalyst may be decomposed through an oxidation-reduction reaction. The filter unit 30 may include a first filter 31 and a second filter 32.

The first filter 31 may be supported by the support part 130 and may be disposed above the extension part 140. In addition, the first filter 31 may be disposed adjacent to the outlet 114 to filter the fluid discharged through the outlet 114.

The second filter 32 may be disposed adjacent to the inlet 113 and may filter the fluid flowing into the housing 110 through the inlet 113.

Although the filter unit 30 has been described as being provided to both the inlet 113 and the outlet 114 in the present specification, this is only an example and the present disclosure is not limited thereto. Accordingly, the filter unit 30 may not be provided on the side of the inlet 113, and may be provided only on the side of the outlet 114. In addition, the filter unit 30 may not be provided on the side of the discharge port 114, and may be provided only on the side of the inlet port 113.

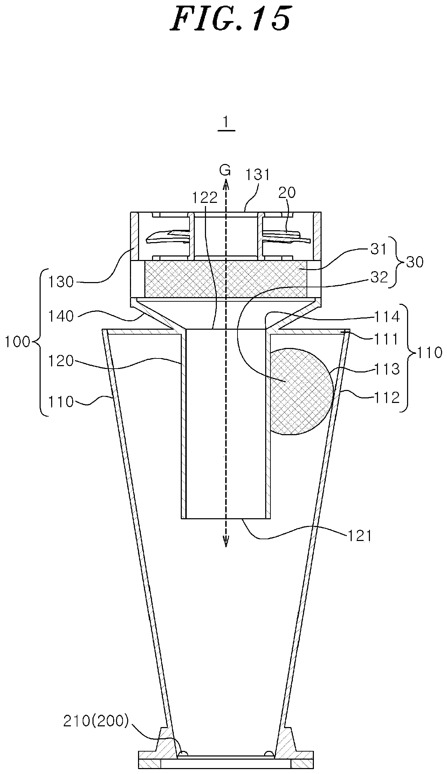

Meanwhile, according to a ninth embodiment of the present disclosure, the light source unit 200 may be disposed on a lower surface of the housing part 110. Hereinafter, the ninth embodiment of the present disclosure will be described with further reference to FIG. 15.

The light source unit 200 may be provided on the lower surface of the housing part 110 to radiate light upward. The light source unit 200 may sterilize the fluid flowing downward from the inlet 113 and the fluid flowing upward from the lower side of the housing part 110. In this case, as the fluid is repeatedly exposed to the light emitted from the light source unit 200, the time during which the fluid is irradiated with the light increases. In addition, the fluid sterilization effect, the fluid deodorizing effect, and the fluid purification effect are increased.

Meanwhile, when the light source unit 200 is disposed on the lower surface of the housing part 110, the light emitted from the light source unit 200 is directed to the outlet 114. In this case, since the filter unit 30 is disposed adjacent to the outlet 114, it is possible to block light from leaving the housing part 110. In addition, the light emitted from the light source unit 200 has an effect of sterilizing the filter unit 30.

In addition, when a light emitting diode is used as the light source 210 of the light source unit 200, an electrode of the light emitting diode may be directly electrically mounted on a substrate. The light emitting diode may be installed on the substrate in an injection-molded lead frame package form for surface mounting, a through-hole mounting form, a bare chip form, or a flip chip form. In addition, the light emitting diode may be installed in a form in which an additional substrate is attached to improve heat dissipation characteristics or electrical characteristics. A connector that connects the light source 210 and a wire may be further provided on the light source unit 200 and the substrate. The wire may be connected to the light source unit 200 through the connector.

The light source 210 may emit light mainly in a direction perpendicular to the surface of the substrate on which the light source 210 is mounted, and a main emission area of light may vary depending on a beam angle. In this case, the beam angle is an angle corresponding to 50% of the maximum amount of light emitted from the light source 210 and means the sum of angles on both sides of a line perpendicular to the center of the light source.

The light emitted from the light source 210 may vary in intensity or amount of light depending on a distance away from the light source 210. That is, the sterilization power may vary depending on to the distance away from the light source 210, and a sterilization light area may be formed.

When the light source unit 200 sterilizes a fluid, the light area by the sterilizing light may be defined as an area to which light emitted from the light source reaches. In this case, at least a portion of the light area may include at least one wavelength conversion material that is excited by light emitted from the light source 210 and converted into a light wavelength within a visible light range. Therefore, the light area defined by the sterilization light emitted from the light source 210 can be visually displayed, so that the sterilization area by the sterilization light can be more easily specified and the state in which the sterilization reaction of the contaminant is occurring can be visually confirmed. In addition, reliability of the fluid treatment module can be further increased by visually checking whether the light source 210 of the light source unit 200 is malfunctioning.

Hereinafter, the operation and effect of the fluid treatment apparatus 1 having the above configurations will be described.

Fluid outside the fluid treatment apparatus 1 may be introduced into the fluid treatment apparatus 1 by the blowing fan 20. The fluid may flow along the flow path part 300 and may flow into the housing part 110 through the inlet 113. For example, the fluid passing through the inlet 113 flows along the flow direction P rather than the central direction C when viewed from above. In this case, the flow rate of the fluid passing through the inlet 113 along the flow direction P is greater than the flow rate flowing along the central direction C. In addition, the fluid passing through the inlet 113 swirls along the inner surface of the housing part 110 and flows downward of the housing part 110. Bacteria and particles included in the fluid flowing along the inner surface of the housing part 110 are adsorbed to the inner surface of the housing part 110 while the fluid swirls.

In addition, some of the fluid passing through the inlet 113 collides with the side surface of the fluid guide part 120 and flows downward. In other words, the fluid that has passed through the inlet 113 does not flow directly into the fluid guide part 120 through the first guide opening 121 but flows to the lower side of the housing part 110. In this case, after flowing downward, the fluid moves up to flow into the fluid guide part 120. In addition, the fluid introduced into the fluid guide part 120 is discharged to the outside through the outlet 114. In this case, the flow path of the fluid introduced into the housing part 110 is lengthened, and the fluid stays inside the housing part 110 for a sufficient time.

Meanwhile, the fluid may be sterilized by being irradiated with light emitted from the light source unit 200 while flowing into the fluid guide part 120 after flowing into the housing part 110 and descending.

In the fluid treatment apparatus 1 according to the embodiments of the present disclosure, the fluid does not directly flow into the fluid guide part 120, but flows downward and then moves up to be discharged through the fluid guide part 120, thereby increasing the time during which the fluid stays inside the housing part 110. In this case, the time during which the fluid is sterilized by the light source unit 200 increases, thereby increasing the fluid sterilization efficiency.

In addition, in the fluid processing device 1, as the fluid passing through the inlet 113 swirls and descends, the time during which the fluid stays inside the housing 110 increases and the number of times the fluid contacts the inner surface of the housing 110 increases. In this case, more bacteria and dust particles included in the fluid can be adsorbed to the inner wall surface of the housing part 110, and the removal efficiency of bacteria and dust particles included in the fluid is increased.

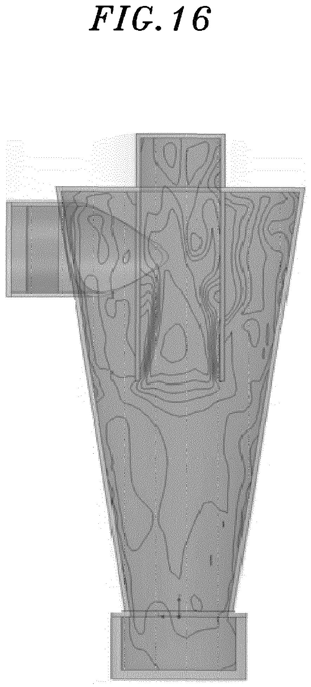

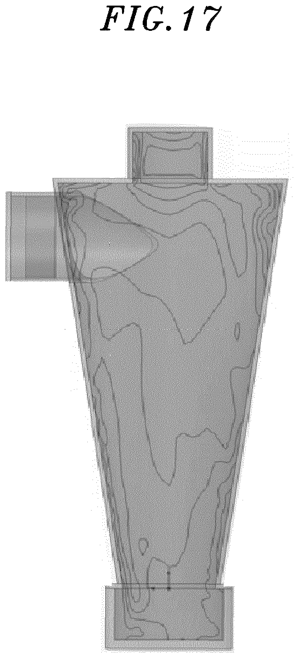

Meanwhile, FIG. 16 is a diagram illustrating a state in which the fluid flows in the fluid treatment apparatus 1 according to the embodiments of the present disclosure. Further, FIG. 17 is a diagram illustrating a state in which the fluid flows in the fluid treatment apparatus 1 where the fluid guide part 120 is omitted.

Referring to FIGS. 16 and 17, in the embodiments of the present disclosure in which the fluid guide part 120 is disposed, the moving distance of the fluid is longer than that of the fluid treatment apparatus 1 in which the fluid guide part 120 is omitted. In other words, the fluid guide part 120 guides the flow of fluid so that the fluid introduced into the inlet 113 turns along the outer surface of the fluid guide part 120 and the inner surface of the housing part 110 to move downward. In this case, the retention time of the fluid flowing in the housing part 110 becomes longer, and the time for the light to be irradiated to the fluid becomes longer. In addition, the fluid sterilizing effect is increased, and the removal efficiency of droplets from the fluid is also increased.

Although the specific embodiments of the present disclosure have been described above, these are merely examples, and the present disclosure is not limited thereto, and should be construed as having the broadest scope according to the technical idea disclosed herein. A person skilled in the art may combine/substitute the disclosed embodiments to implement a pattern of a shape that is not disclosed, but it also does not depart from the scope of the present disclosure. In addition, those skilled in the art can easily change or modify the disclosed embodiments based on the present specification, and it is clear that such changes or modifications also belong to the scope of the present disclosure.

## Claims

1. A fluid treatment module comprising:
a housing part having a space for fluid to flow in an up-down direction therein and having an inlet through which the fluid is introduced into the housing part and an outlet through which the fluid in the housing part is discharged to an outside thereof;
a light source unit that irradiates light to the fluid flowing in the housing part; and
a fluid guide part that guides the flow of the fluid in the housing part, one end of the fluid guide part being opened in a direction different from a direction in which the inlet is opened, and the other end thereof communicating with the outlet,
wherein the inlet is oriented so that the fluid passing through the inlet flows along a direction different from a direction from a center of the inlet to a center of the housing part when viewed from above.

2. The fluid treatment module of claim 1, wherein the outlet is formed in an upper surface of the housing part,
the inlet is formed in a side surface of the housing part, and
the fluid guide part extends downward from the outlet.

3. The fluid treatment module of claim 2, wherein the fluid guide part has at the one end a guide opening opened downward, and the guide opening is disposed at a position higher than 1/2 of a length of the housing part in the up-down direction and lower than or equal to the inlet.

4. The fluid treatment module of claim 1, further comprising:
a flow path part providing a passage through which the fluid flows and extending from the inlet so that the fluid flows into the housing part,
wherein when viewed from above, the flow path part extends in a flow path direction, which is a direction different from the direction from the center of the inlet to the center of the housing part.

5. The fluid treatment module of claim 4, wherein when viewed in the flow path direction, at least a portion of the inlet overlaps the fluid guide part.

6. The fluid treatment module of claim 4, wherein when viewed in the flow path direction, the inlet is disposed to be horizontally spaced apart from an imaginary guide centerline extending in the up-down direction while passing through a center of the fluid guide part.

7. The fluid treatment module of claim 6, wherein when viewed from above, the fluid guide part and the housing part are arranged concentrically.

8. The fluid treatment module of claim 1, wherein the housing part is provided to have a circular or elliptical cross section, a side surface thereof is extended to have a predetermined inclination with respect to a horizontal direction, and the housing part is provided so that an upper cross section thereof has a larger cross-sectional area than that of a lower cross section.

9. The fluid treatment module of claim 1, further comprising:
an adsorption auxiliary part provided on at least one of an inner surface of the housing part and an outer surface of the fluid guide part,
wherein the adsorption auxiliary part extends along a circumferential direction of the at least one surface to protrude from or be depressed into the at least one surface.

10. The fluid treatment module of claim 1, further comprising:
a photocatalyst part provided on at least one of an inner surface of the housing part and an outer surface of the fluid guide part,
wherein the photocatalyst part includes at least one of titanium oxide, zinc oxide, and tin oxide that causes a catalytic reaction by the light irradiated from the light source unit.

11. The fluid treatment module of claim 1, further comprising:
a reflecting part provided on at least one of an inner surface of the housing part and an outer surface of the fluid guide part,
wherein the reflecting part includes a reflective material that reflects the light irradiated from the light source unit.

12. The fluid treatment module of claim 1, further comprising:
an antibacterial part provided on at least one of an inner surface of the housing part and an outer surface of the fluid guide part,
wherein the antibacterial part includes an antibacterial material for secondary sterilization of the fluid.

13. The fluid treatment module of claim 1, wherein the fluid guide part includes a transmissive material that transmits the light.

14. The fluid treatment module of claim 1, wherein the light source unit is disposed between an inner surface of the housing part and an outer surface of the fluid guide part.

15. A fluid treatment module comprising:
a housing part having a space for fluid to flow in an up-down direction therein and having an inlet through which the fluid is introduced into the housing part and an outlet through which the fluid in the housing part is discharged to an outside thereof;
a light source unit that irradiates light to the fluid flowing in the housing part; and
a fluid guide part that guides the flow of the fluid in the housing part, one end of the fluid guide part being opened in a direction different from a direction in which the inlet is opened, and the other end thereof communicating with the outlet,
wherein the housing part is provided to have a smaller cross-sectional area from upper side to lower side, and the housing part is configured such that the fluid passing through the inlet swirls along a circumferential direction of the housing part and flows downward.

16. The fluid treatment module of claim 15, wherein the outlet is formed in an upper surface of the housing part,
the inlet is formed in a side surface of the housing part, and
the fluid guide part extends downward from the outlet.

17. A fluid treatment apparatus comprising:
a fluid treatment module for sterilizing fluid; and
a blowing fan for providing a blowing force to cause the fluid to flow into the fluid treatment module,
wherein the fluid treatment module includes:
a housing part having a space for fluid to flow in an up-down direction therein and having an inlet through which the fluid is introduced into the housing part and an outlet through which the fluid in the housing part is discharged to an outside thereof;
a light source unit that irradiates light to the fluid flowing in the housing part; and
a fluid guide part that guides the flow of the fluid in the housing part, one end of the fluid guide part being opened in a direction different from a direction in which the inlet is opened, and the other end thereof communicating with the outlet,
wherein the inlet is oriented so that the fluid passing through the inlet flows along a direction different from a direction from a center of the inlet to a center of the housing part when viewed from above.

18. The fluid treatment apparatus of claim 17, wherein the fluid treatment module further includes an extension part disposed between the housing part and the blowing fan and guiding a flow of the fluid discharged from the outlet, and
the extension part has one end communicating with the outlet and has a shape in which a cross-sectional area becomes wider from the one end to the other end.

19. The fluid treatment apparatus of claim 18, further comprising:
a filter unit disposed between the extension part and the blowing fan to filter the fluid.

20. The fluid treatment apparatus of claim 17, further comprising:
a filter unit that filters the fluid,
wherein the filter unit includes a plurality of filters disposed adjacent to the inlet and the outlet.
